(19) Europäisches Patentamt
European Patent Office
Office européen des brevets

(11) Veröffentlichungsnummer: **0 356 947**
**A2**

# (12) EUROPÄISCHE PATENTANMELDUNG

(21) Anmeldenummer: **89115767.9**

(51) Int. Cl.5: **C07C 227/12 , C07C 231/06**

(22) Anmeldetag: **26.08.89**

(30) Priorität: **02.09.88 DE 3829829**

(43) Veröffentlichungstag der Anmeldung:
**07.03.90 Patentblatt 90/10**

(84) Benannte Vertragsstaaten:
**BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Widder, Rudi, Dr.**
**In der Taesch 7**
**D-6906 Leimen(DE)**
Erfinder: **Gousetis, Charalampos, Dr.**
**Carl-Bosch-Strasse 98**
**D-6700 Ludwigshafen(DE)**
Erfinder: **Perner, Johannes, Dr.**
**Ginsterweg 4**
**D-6730 Neustadt(DE)**
Erfinder: **Wolf, Helmut**
**Im Zollstock 6**
**D-6733 Hassloch(DE)**
Erfinder: **Bochnitschek, Werner, Dr.**
**Sinsheimer Strasse 49**
**D-6700 Ludwigshafen(DE)**
Erfinder: **Oftring, Alfred, Dr.**
**Im Roehrich 49**
**D-6702 Bad Duerkheim(DE)**

## (54) Verfahren zur Herstellung des Trinatriumsalzes von Isoserin-N,N-diessigsäure.

(57) Herstellung des Trinatriumsalzes von Isoserin-N,N-diessigsäure (I) durch Umsetzung von Acrylnitril mit wäßrigem Wasserstoffperoxid zu Glycidsäureamid (II) und weitere Umsetzung von II mit dem Dinatriumsalz von Iminodiessigsäure (III) zu I, indem man das Verfahren kontinuierlich durchführt und hierbei nacheinander

a) Acrylnitril und wäßriges Wasserstoffperoxid bei pH 7-8 und 30-60 °C miteinander reagieren läßt,

b) nicht umgesetztes Acrylnitril destillativ entfernt,

c) das Reaktionsgemisch über einen festen Wasserstoffperoxid-Zersetzungskontakt leitet,

d) die so erhaltene wäßrige Lösung von II mit III bei pH 7-9 und 40-90 °C umsetzt,

e) die resultierende Lösung mit einer basischen Natriumverbindung bei pH 9-14 und 60-95 °C reagieren läßt,

f) entstandenes Ammoniak destillativ entfernt und

g) die so erhaltene wäßrige Lösung von I gewünschtenfalls in an sich bekannter Weise auf festes I aufarbeitet.

EP 0 356 947 A2

## Verfahren zur Herstellung des Trinatriumsalzes von Isoserin-N,N-diessigsäure

Die vorliegende Erfindung betrifft ein verbessertes Verfahren zur Herstellung des Trinatriumsalzes von Isoserin-N,N-diessigsäure der Formel I

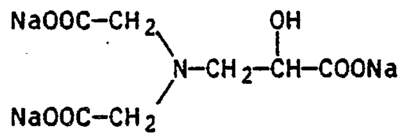

I

durch Umsetzung von Acrylnitril mit wäßrigem Wasserstoffperoxid zu Glycidsäureamid der Formel II

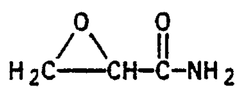

II

und weitere Umsetzung von II mit dem Dinatriumsalz von Iminodiessigsäure der Formel III

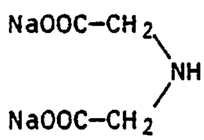

III

zu I.

Die ältere deutsche Patentanmeldung P 37 12 330 (1) lehrt ein Verfahren zur Herstellung des Trinatriumsalzes I, das als Komplexbildner, Gerüststoff und Bleichmittelstabilisator in Wasch- und Reinigungsmitteln Verwendung finden kann, durch Zugabe von Glycidsäureamid (II) zu einer wäßrigen Lösung von Iminodiessigsäure in der doppelten molaren Menge Natronlauge und anschließende Hydrolyse der Amidgruppe durch Erhitzen in Natronlauge.

Die Herstellung von II erfolgt gemäß DE-A 19 04 077 (2) durch Umsetzung von Acrylnitril mit wäßrigem Wasserstoffperoxid in einem engen pH-Bereich von beispielsweise 7,2 bis 7,4. Danach wird nicht umgesetztes Acrylnitril abdestilliert und nicht verbrauchtes Peroxid mit Katalytischen Mengen einer Manganverbindung zerstört.

Die in (1) und (2) angeführten Syntheseverfahren sind diskontinuierlicher Natur. Nachteile bei diesen Verfahren sind die nur schwer kontrollierbare Wärmeabfuhr, insbesondere bei der Reaktion von Acrylnitril mit Wasserstoffperoxid zu II, sowie die Schwierigkeit, den pH-Wert konstant zu halten. Außerdem kann bei der in (2) beschriebenen Vorgehensweise beim Abdestillieren des nicht umgesetzten Acrylnitrils und beim anschließenden Zerstören des restlichen Peroxids mit Manganverbindungen ein merkbarer Ausbeuteverlust an II durch die thermische Belastung auftreten.

Der Erfindung lag daher die Aufgabe zugrunde, der Technik ein in der Reaktionsführung sicher beherrschbares Verfahren zur Herstellung von I mit hohen Raum-Zeit-Ausbeuten zur Verfügung zu stellen.

Demgemäß wurde ein Verfahren zur Herstellung des Trinatriumsalzes I durch Umsetzung von Acrylnitril mit wäßrigem Wasserstoffperoxid zu Glycidsäureamid (II) und weitere Umsetzung von II mit dem Dinatriumsalz III zu I gefunden, welches dadurch gekennzeichnet ist, daß man das Verfahren kontinuierlich durchführt und hierbei nacheinander

a) Acrylnitril und wäßriges Wasserstoffperoxid bei einem pH-Wert von 7 bis 8 und einer Temperatur von 30 bis 60 °C miteinander reagieren läßt,

b) nicht umgesetztes Acrylnitril destillativ entfernt,

c) das Reaktionsgemisch über einen festen Wasserstoffperoxid-Zersetzungskontakt leitet,

d) die so erhaltene wäßrige Lösung von II mit III bei einem pH-Wert von 7 bis 9 und einer Temperatur von 40 bis 90 °C umsetzt,

e) die resultierende Lösung mit einer basischen Natriumverbindung bei einem pH-Wert von 9 bis 14 und einer Temperatur von 60 bis 95 °C reagieren läßt,

f) entstandenes Ammoniak destillativ entfernt und

g) die so erhaltene wäßrige Lösung von I gewünschtenfalls in an sich bekannter Weise auf festes I aufarbeitet.

Die Reaktionsschritte a), d) und e) des erfindungsgemäßen Verfahrens werden vorzugsweise in einer Rührkesselkaskade durchgeführt, wobei für jeden der drei Schritte mindestens zwei Rührkessel erforderlich sind (als Beispiel siehe Figur). Es sind aber auch Ausführungsformen möglich, bei denen die Rührkesselelemente der Anlage teilweise oder ganz durch Rohrreaktoren ersetzt sind.

Für die nacheinander auszuführenden Einzelschritte a) bis g) gelten folgende Empfehlungen:

a) Acrylnitril und eine wäßrige Wasserstoffperoxidlösung, deren Gehalt im Bereich von 3 bis 50 Gew.-%, vorzugsweise 10 bis 35 Gew.-%, liegt, werden im molaren Verhältnis von 1 : 0,6 bis 1 : 1,5, vorzugsweise 1 : 1 bis 1 : 1,2, in einem Rührkessel bei einem pH-Wert von 7 bis 8, vorzugsweise 7,3 bis 7,7, und einer Temperatur von 30 bis 60 °C, vorzugsweise 45 bis 55 °C, miteinander zur Reaktion gebracht. Die mittlere Verweilzeit im Rührkessel beträgt üblicherweise 30 bis 45 Minuten. Zur Konstanthaltung des pH-Wertes und der Temperatur wird 5 bis 50 gew. -%ige, vorzugsweise 10 bis 20 gew. -%ige Natronlauge zudosiert In einem bis drei, vorzugsweise einem nachgeschalteten Rührkessel wird bei den gleichen Bedingungen bezüglich pH-Wert und Temperatur wie im ersten Rührkessel die Reaktionslösung nachvermischt. Die mittlere Verweilzeit im zweiten Rührkessel beträgt üblicherweise 15 bis 25 Minuten.

b) Das bei a) nicht umgesetzte Acrylnitril, dessen Anteil normalerweise 10 bis 30 Gew.-% der eingesetzten Menge beträgt, wird in einer Destillationskolonne, die vorzugsweise nach dem Dünnschichtverdampfer-Prinzip arbeitet, abgetrennt. Die Destillationskolonne wird vorzugsweise bei der gleichen Temperatur wie bei a) und bei einem Druck von vorteilhafterweise 120 bis 160 mbar betrieben. Zusammen mit dem Acrylnitril wird in der Regel eine geringe Menge Wasser abdestilliert. Nach der Destillation beträgt der Restgehalt an Acrylnitril in der Reaktionslösung normalerweise 0,1 bis 0,5 Gew.-% Das abgetrennte Acrylnitril kann vorteilhafterweise wieder in den Schritt a) zurückgeführt werden.

c) Die Reaktionslösung wird zur Zersetzung von noch vorhandenem Wasserstoffperoxid über einen festen Kontakt, vorzugsweise aus Aktivkohle, geleitet. Es können aber auch andere feste Materialien zur Zerstörung des Wasserstoffperoxids, beispielsweise Zeolithe oder wasserunlösliche Mangan-, Blei- oder Vanadiumverbindungen, verwendet werden. Die Reihenfolge der Schritte b) und c) kann vertauscht werden, jedoch wird es bevorzugt, zuerst nicht umgesetztes Acrylnitril abzudestillieren.

d) Die erhaltene normalerweise 15 bis 30 gew. -%ige wäßrige Lösung von Glycidsäureamid (II) wird mit dem Dinatriumsalz von Iminodiessigsäure (III) in Form einer üblicherweise 20 bis 30 gew.-%igen wäßrigen Lösung im molaren Verhältnis von 1 : 0,9 bis 1 : 1,1, vorzugsweise 1 : 1, in einem Rührkessel bei einer Temperatur von 40 bis 90 °C, vorzugsweise 55 bis 80 °C, umgesetzt. In einem bis drei, vorzugsweise zwei nachgeschalteten Rührkesseln wird die Reaktionslösung nachvermischt. Dabei kann vorteilhafterweise ein Temperaturgradient eingestellt werden, so daß im letzten Rührkessel von d) eine um 10 bis 25 °C, vorzugsweise 15 bis 20 °C höhere Temperatur erreicht wird. Der für den ersten Rührkessel von d) angegebene Temperaturbereich gilt für alle Kessel einschließlich des letzten. Beim Durchlaufen aller Rührkessel von d) stellt sich für die Reaktionslösung ein pH-Wert von 7 bis 9, vorzugsweise 7,5 bis 8,5, ein. Die mittlere Verweilzeit im ersten Rührkessel beträgt üblicherweise 15 bis 30 Minuten und steigert sich bis auf üblicherweise 45 bis 90 Minuten für den letzten Rührkessel.

e) Die erhaltene wäßrige Lösung des Dinatriumsalzes von 2-Hydroxy-3-aminopropionsäureamid-N,N-diessigsäure wird mit einer 20 bis 50 gew. -%igen, vorzugsweise 40 bis 50 gew. -%igen Lösung einer basischen Natriumverbindung, z.B. wäßriger Natriumcarbonatlösung oder vorzugsweise Natronlauge, in molarem Verhältnis von 1 : 1 bis 1 : 1,7, vorzugsweise 1 : 1,3 bis 1 : 1,4, in einem Rührkessel bei einer Temperatur von 60 bis 95 °C, vorzugsweise 70 bis 85 °C, und einem pH-Wert von 9 bis 14, vorzugsweise 11 bis 12,5, zum Endprodukt I verseift. In einem bis drei, vorzugsweise einem nachgeschalteten Rührkessel wird bei den gleichen Bedingungen bezüglich Temperatur und pH-Wert wie im ersten Rührkessel von e) die Reaktionslösung nachvermischt. Dabei kann vorteilhafterweise ein Druckgradient eingestellt werden, so daß im letzten Rührkessel von e) ein um 200 bis 500 mbar, vorzugsweise 250 bis 400 mbar, gegenüber dem bei Normaldruck betriebenen ersten Rührkessel verminderter Druck herrscht. Die mittlere Verweilzeit beträgt für alle Stufen von e) üblicherweise jeweils 50 bis 100 Minuten. Der größte Teil des bei der Verseifung gebildeten Ammoniaks wird direkt aus Schritt e) gasförmig abgeführt.

f) Die restliche Menge des bei e) entstandenen Ammoniaks wird als Mischung mit Wasser in einer Stripp- oder Destillationskolonne, die vorzugsweise nach dem Dünnschichtverdampfer-Prinzip arbeitet, abgetrennt. Die Destillationskolonne wird vorzugsweise bei der gleichen Temperatur wie bei e) und bei einem Druck von 200 bis 700 mbar, vorzugsweise 400 bis 600 mbar, betrieben. Es verbleibt eine in der Regel 10 bis 20 gew. -%ige wäßrige Lösung des Trinatriumsalzes I mit einem Gesamtfeststoffgehalt von üblicherweise 20 bis 40 Gew.-% und einem pH-Wert von normalerweise 11,5 bis 13.

g) Die so erhaltene wäßrige Lösung von I kann in an sich bekannter Weise, beispielsweise durch

Sprüh- oder Gefriertrocknung, Kristallisation, Fällung oder Eindampfen, auf festes I aufgearbeitet werden.

Der Vorzug des erfindungsgemäßen Verfahrens liegt in der Kombination von mehreren Einzelvorteilen, deren Zusammenwirken zu einem hervorragenden Gesamtergebnis führt. Durch das kontinuierliche Zudosieren von Acryl nitril und Wasserstoffperoxid bei Schritt a) und den dadurch bedingten konstanten Reaktionsverlauf bereitet die Abfuhr der Reaktionswärme der stark exothermen Reaktion keine Schwierigkeiten. Der in Abhängigkeit zur Reaktionstemperatur stehende pH-Wert bei Schritt a) läßt sich somit mühelos auf seinem eng begrenzten Sollwert halten. Die Herstellung von Glycidsäureamid (II) verläuft so wesentlich reproduzierbarer als nach dem in (2) beschriebenen diskontinuierlichen Verfahren.

Die destillative Entfernung von nicht umgesetztem Acrylnitril in Schritt b), vorzugsweise mit einem Dünnschichtverdampfer, erfolgt nahezu vollständig. Durch die Rückfuhrmöglichkeit des abdestillierten Acrylnitrils in den Herstellungsprozeß kann die Ausbeute an II wesentlich erhöht werden.

Durch die vollständige Zerstörung von noch vorhandenem Wasserstoffperoxid durch den Feststoffkontakt des Schrittes c) wird die Möglichkeit von Oxidations- und/oder Hydrolyse-Nebenreaktionen in den weiteren Schritten des erfindungsgemäßen Verfahrens ausgeschlossen. Außerdem ist die vollständige Entfernung von Wasserstoffperoxid-Resten und von eventuell vorhandenen Spuren von Hydroperoxiden wegen der Gefahr von Explosionen auch von sicherheitstechnischem Interesse.

Da die wäßrige Lösung von II nicht lagerstabil ist und sich besonders bei thermischer Belastung rasch zersetzt, sind die schonend ablaufenden Destillations- und $H_2O_2$-Zersetzungsschritte b) bzw. c) von ausschlaggebender Bedeutung für die Reinheit und den Gehalt der in Schritt d) eingesetzten Lösung. Die sofortige weitere Umsetzung von II, bedingt durch die kontinuierliche Vorgehensweise, ist ein wesentlicher Vorteil des erfindungsgemäßen Verfahrens, da hierbei Zersetzungsreaktionen von II, die zu nicht gewünschten Nebenprodukten und zu Ausbeuteeinbußen am Endprodukt I führen, weitgehend vermieden werden.

Durch die kontinuierliche Arbeitsweise der Umsetzung von II mit III bei Schritt d) und der Carbonsäureamid-Verseifung bei Schritt e) laufen die Reaktionen vollständiger ab und sind vor allem besser zu kontrollieren. Die Entfernung der Restmengen des bei e) entstandenen Ammoniaks im Schritt f), vorzugsweise durch Dünnschichtverdampfung, stellt wie bei b) ein schonendes und effizientes Trennverfahren dar.

Für die Gesamtumsetzung von Acrylnitril zum Endprodukt I ist anzumerken, daß es sich hier um ein Verfahren mit einer hohen Raum-Zeit-Ausbeute handelt.

Beispiel

Das folgende Beispiel soll das erfindungsgemäße Verfahren anhand der Versuchsanlage, die in der Figur skizziert ist, näher erläutern:

a) Pro Stunde wurden 255 g (4,8 mol) Acrylnitril (AN) und 1200 g einer 15 gew. -%igen wäßrigen Wasserstoffperoxid-Lösung (entsprechend 5,3 mol $H_2O_2$) zusammen mit stündlich 110 g einer 10 gew.-%igen Natronlauge (entsprechend 0,28 mol NaOH) bei 50 °C in den Rührkessel R1 (Volumen 1,0 l) so eindosiert, daß der pH-Wert zwischen 7,4 und 7,5 gehalten wurde. Die Reaktionsmischung gelangte nach einer Verweilzeit von 38 min in den Rührkessel R2 (Volumen 0,5 l), wo weitere 60 g der 10 gew. -%igen Natronlauge (entsprechend 0,15 mol NaOH) pro Stunde zur Konstanthaltung des genannten pH-Wertes eingetragen wurden. Die Temperatur im Rührkessel R2 betrug 50 °C, die Verweilzeit 18 min.

b) Aus der Reaktionsmischung wurden im Sambay-Dünnschichtverdampfer D1, der bei 50 °C und 140 mbar betrieben wurde, stündlich 58 g (1,1 mol) nicht umgesetztes Acrylnitril sowie 35 g Wasser abdestilliert. Nach Verflüssigung des Destillats im Kondensator K und Phasentrennung im Scheidetrichter S wurden pro Stunde 55 g Acrylnitril (AN) wieder in den Schritt a) zurückgeführt. Das in S als wäßrige Phase anfallende Gemisch aus 92 Gew.-% Wasser und 8 Gew.-% Acrylnitril (AN) wurde zum Einstellen der in a) benötigten Natronlauge auf 10 Gew.-% verwendet.

c) Die Reaktionsmischung wurde über den Kontakt Z mit Aktivkohle in Granulatform geleitet, um noch vorhandenes Wasserstoffperoxid und evtl. Spuren von Hydroperoxiden zu zerstören. Die Menge an Aktivkohle betrug 150 g, ihre geschätzte freie Oberfläche 500 $m^2$. Man erhielt so pro Stunde 1550 g wäßrige Glycidsäureamidlösung mit einem Gehalt von 19 Gew.-% (entsprechend einer Ausbeute von 90 %)und einem Restgehalt an Acrylnitril von 0,1 Gew.-%; $H_2O_2$ und/oder andere Peroxide ließen sich analytisch nicht mehr nachweisen.

d) Die erhaltene Glycidsäureamidlösung (II) wurde mit stündlich 2360 g einer 25 gew. -%igen Lösung des Dinatriumsalzes von Iminodiessigsäure (III) (entsprechend 3,33 mol wasserfreiem Reagenz) bei 55 °C im Rührkessel R3 (Volumen 1,0 l) vermischt. Nach einer Verweilzeit von 20 min gelangte die Reaktionslösung in den Rührkessel R4 (Volumen 2,0 l) und blieb dort 40 min bei 65 °C. Danach gelangte

die Lösung in den Rührkessel R5 (Volumen 3,0 l), dessen Innentemperatur auf 75 °C eingestellt war, und blieb dort 60 min. Der pH-Wert stellte sich im Rührkessel R5 auf 7,8 bis 8,2 ein.

e) Die Reaktionslösung wurde im Rührkessel R6 (Volumen 4,0 l) pro Stunde mit 346 g einer 50 gew.-%igen Natronlauge (entsprechend 4, 33 mol NaOH) bei 80 °C und einem pH-Wert von 11,8 bis 12,5 zum Endprodukt verseift. Nach einer Verweilzeit von 80 min gelangte die Reaktionslösung in den Rührkessel R7 (Volumen 4,0 l) und wurde dort bei gleicher Temperatur, gleichem pH-Wert und gleicher Verweilzeit wie im Rührkessel R6 bei einem vermindertem Druck von 700 mbar nachvermischt. Der größte Teil des bei der Verseifung entstandenen Ammoniaks wurde direkt aus den Rührkesseln R6 und R7 gasförmig abgeführt.

f) Zur destillativen Entfernung des restlichen Ammoniaks wurden im Sambay-Dünnschichtverdampfer D2 bei 80 °C und 500 mbar stündlich 500 g einer Ammoniak/Wasser-Mischung im Gewichtsverhältnis von 1 : 70 abgetrennt. Man erhielt als Endprodukt stündlich 3750 g einer wäßrigen Lösung des Trinatriumsalzes von Isoserin-N,N-diessigsäure (I) mit einem Gehalt von 16 Gew.-% (entsprechend einer Ausbeute von 63 %, bezogen auf in Schritt d) eingesetztes II), einem Gesamtfeststoffgehalt von 30 Gew.-% und einem pH-Wert von 12,5.

## Ansprüche

1. Verfahren zur Herstellung des Trinatriumsalzes von Isoserin-N,N-diessigsäure der Formel I

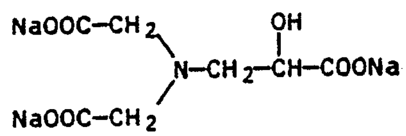

I

durch Umsetzung von Acrylnitril mit wäßrigem Wasserstoffperoxid zu Glycidsäureamid der Formel II

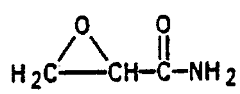

II

und weitere Umsetzung von II mit dem Dinatriumsalz von Iminodiessigsäure der Formel III

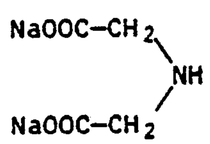

III

zu I, dadurch gekennzeichnet, daß man das Verfahren kontinuierlich durchführt und hierbei nacheinander

a) Acrylnitril und wäßriges Wasserstoffperoxid bei einem pH-Wert von 7 bis 8 und einer Temperatur von 30 bis 60 °C miteinander reagieren läßt,

b) nicht umgesetztes Acrylnitril destillativ entfernt,

c) das Reaktionsgemisch über einen festen Wasserstoffperoxid-Zersetzungskontakt leitet,

d) die so erhaltene wäßrige Lösung von II mit III bei einem pH-Wert von 7 bis 9 und einer Temperatur von 40 bis 90 °C umsetzt,

e) die resultierende Lösung mit einer basischen Natriumverbindung bei einem pH-Wert von 9 bis 14 und einer Temperatur von 60 bis 95 °C reagieren läßt,

f) entstandenes Ammoniak destillativ entfernt und

g) die so erhaltene wäßrige Lösung von I gewünschtenfalls in an sich bekannter Weise auf festes I aufarbeitet.

2. Verfahren nach Anspruch 1, dadurch gekennzeichnet, daß man die Reaktionsschritte a), d) und e) in einer Rührkesselkaskade durchführt, wobei für jeden der drei Schritte mindestens zwei Rührkessel erforderlich sind.

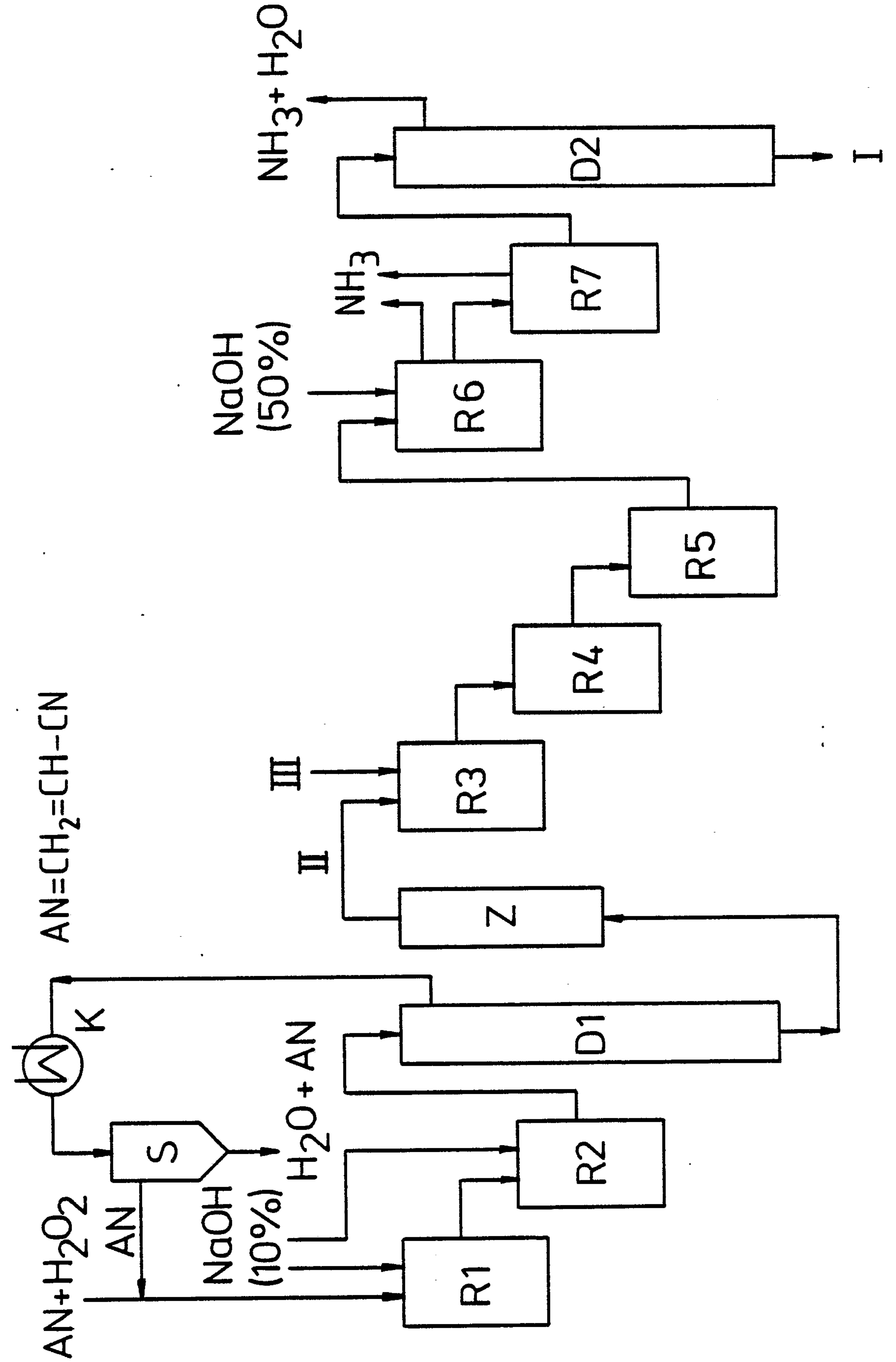
AN+H2O2
AN
NaOH (10%)
S
H2O + AN
K
R1
R2
D1
Z
II
III
R3
R4
R5
NaOH (50%)
R6
NH3
R7
D2
NH3 + H2O
I
AN=CH2=CH-CN